# EUROPÄISCHE PATENTSCHRIFT

(11) **EP 1 185 384 B1**
(45) Veröffentlichungstag und Bekanntmachung des Hinweises auf die Patenterteilung: **22.09.2004**
(21) Anmeldenummer: 00931028.5
(22) Anmeldetag: 20.04.2000
(51) Int. Cl.: B09B 3/00, C12M 1/14

(54) **VERFAHREN UND VORRICHTUNG ZUR BEHANDLUNG VON STRUKTURFREIEN ODER STRUKTURARMEN BIOABFÄLLEN**
METHOD AND DEVICE FOR TREATING UNSTRUCTURED OR LOOSELY STRUCTURED ORGANIC WASTE
PROCEDE ET DISPOSITIF POUR LE TRAITEMENT DE DECHETS BIOLOGIQUES EXEMPTS DE STRUCTURES OU PAUVRES EN STRUCTURES

(30) Priorität: 23.06.1999 DE 19928663
(43) Veröffentlichungstag der Anmeldung: 13.03.2002
(73) Patentinhaber: BEG BIOENERGIE GESELLSCHAFT MBH, 45127 Essen (DE)
(72) Erfinder: KANITZ, Jürgen, D-44805 Bremen (DE)
(74) Vertreter: Vomberg, Friedhelm, Dipl.-Phys.
(86) Internationale Anmeldenummer: PCT/DE2000/001286
(87) Internationale Veröffentlichungsnummer: WO 2001/000341

(56) Entgegenhaltungen:
- EP-A- 0 253 774
- EP-A- 0 647 605
- EP-A- 0 679 719
- EP-A- 0 866 042
- DE-C- 3 821 028

## Beschreibung

Die Erfindung betrifft ein Verfahren zur Behandlung von strukturfreien oder strukturarmen Bioabfällen, nämlich Speiseresten und/oder -rückständen oder -abfällen aus Haushalten, der Gastronomie sowie der Lebensmittelindustrie, die nach ihrer Zerkleinerung und Homogenisierung einer weitgehend aeroben Hydrolyse unterworfen und danach einer anaeroben Methanisierung zugeführt werden, bei der das entstehende Biogas abgezogen wird. Die Erfindung betrifft ferner eine Vorrichtung zur Durchführung des Verfahrens mit einer Zerkleinerungsvorrichtung, einer Mischvorrichtung, einem beluftbaren Hydrolysebehälter, einem Biogasreaktor, einem Absetzbehälter und jeweiligen Materialfördereinrichtungen zur Überführung des Bioabfalls in die nächste Stufe bzw. Rückführung in eine vorherige Stufe.

In der EP 0 773 914 B1 wird ein Verfahren und eine Vorrichtung zur Behandlung von organischen Bioreststoffen beschrieben, die aus kommunalem und/oder gewerblichen Abfallstoffen einschließlich roher und/oder gekochter Speisereste, landwirtschaftlicher Abfälle, insbesondere Tierexkremente und/oder pflanzlichen Bestandteilen bestehen, die unter intensivem Mischen und Homogenisieren durch Pressen in eine flüssige Fraktion mit in Wasser gelösten oder lösbaren C-haltigen organischen Bestandteilen und in aus den verbleibenden Feststoffen bestehenden festen Fraktionen getrennt werden, wobei die organischen Bio-Reststoffe vor dem Pressen über oberhalb und unterhalb eines schräg liegenden Tisches oder Tischblechen jeweils parallel hierzu angeordneten Förderschnecken in einen geschlossenen Kreislauf geführt werden, in dessen Verlauf die Bio-Reststoffe aufgelockert und die aufgelockerten Bio-Reststoffe befeuchtet und/oder belüftet werden. Die Auflockerung der Bio-Reststoffe wird durch eine Erhöhung der Fördergeschwindigkeit in einem Teil des Kreislaufs durchgeführt, wozu die betreffende Vorrichtung Förderschnecken aufweist, von denen die aufwärtsfördernden Schnecken unterschiedliche Steigungen der Schneckenwendel aufweisen.

Nachfolgend werden die derart vorbehandelten Bioabfälle einem Anaerob-Reaktor zur Methanisierung zugeführt.

Das vorbeschriebenen Verfahren und die hierzu verwendete Vorrichtung ist für heterogenen Bioabfall ausgelegt, jedoch nicht für Monochargen geeignet, die aus Speiseresten oder aus Rückständen aus Abfällen aus dem Bereich der Lebensmittelindustrie bestehen. Solches Bio-Material weist keinerlei Struktur auf und kann somit nicht im Schneckentrieb gefördert werden. Insbesondere ist die mit einer Förderschnecke arbeitende Vorrichtung nicht für die Behandlung von Speiseresten aus Großküchen, Restaurantbetrieben, Schnellrestaurants oder für Abfälle aus der Lebensmittelindustrie, aus Schlachthöfen oder Brauereien etc. geeignet.

Abhilfe können lediglich solche Verfahren und Vorrichtungen schaffen, die dazu geeignet sind, die Bioabfälle vor der aeroben Behandlung aufzuschließen und zu zerkleinern, anschließend zu hydrolysieren und zu methanisieren.

Ein solches Verfahren, das allerdings auf die Verwertung von Tierkörpern beschränkt ist, wird beispielsweise in der DE 196 23 163 A1 beschrieben. Die aus Tierkörpern bestehenden Abfälle werden zerkleinert, durch Aufschluß vorbehandelt, bei Temperaturen von 18°C bis 40°C hydrolysiert, so daß das Hydrolysat einen pH-Wert von 3 bis 5 aufweist, bei Temperaturen von 50°C bis 60°C bei einem pH-Wert von 6,5 bis 8,5 methanisiert und anschließend hygienisiert, wobei Biogas und ein Flüssigdünger entstehen. Der Aufschluß kann thermisch, chemisch oder biochemisch erfolgen. Die Hydrolyse soll unter Belüftung semianaerob durchgeführt werden, wobei die Belüftungsrate in Abhängigkeit von den Fettsäurespektren gewählt wird, die wiederum vom Anteil der unterschiedlichen Substanzen in der Zuführung wie Fette, Zucker, Stärke oder Proteine abhängig ist. Das Bio-Material verweilt in der Hydrolyse 3 bis 5 Tage, bevor es in die 10 bis 20 Tage andauernde Methanisierung überführt wird. Ein ähnliches Verfahren beschreibt die DE 196 28 521 A1, bei dem die aus Tierkörperteilen oder von Tieren stammenden Erzeugnisse in einer Zerkleinerungsstufe zuerst mittels eines Brechers grob zerkleinert und in einem nachgeschalteten Feinzerkleinerer feinzerkleinert und homogenisiert sollen. Anschließend wird das Produkt sterilisiert und der erhaltene Produktbrei abgekühlt, unter ständiger Bewegung hydrolysiert und schließlich unter anaeroben Bedingungen in einem Bio-Reaktor zu Biogas umgesetzt. Feinzerkleinerung soll die Ausgangssubstanz zu Korngrößen zwischen 20 bis 5 mm überführen.

Ferner beschreibt die EP-A-866 042 ein Verfahren zur Behandlung von Bioabfällen, bestehend aus strukturarmen Bioabfällen, wie nativ organischen Bestandteilen des Hausmülls aus getrennten Sammlungen, Großküchenabfällen, Abfällen aus lebensmittelverarbeitenden Betrieben sowie vorgenannten Stoffen ähnlichen organischen Rückständen, bei dem die aufgegebenen Bioabfälle zerkleinert, gemischt und mit Wasser in Form einer Suspension nach Absiebung von Störstoffen einer Hydrolyse und danach einer Fermentation (Methanisierung) unterzogen werden, woraus ein verbleibender Gärschlamm entwässert und einer Kompostierung zugeführt wird, wobei nach der Hydrolyse und vor der Fermentation eine Hygienisierung durchgeführt werden soll. Um die Wirksamkeit der Hygienisierung zu optimieren, wird vorgeschlagen, die Bioabfälle vor der Hydrolyse auf eine Größe von maximal 1 cm, vorzugsweise ≤ 0,7 cm, zu zerkleinern, wozu sich ein Mazerator, d.h. eine Zerkleinerungsvorrichtung mit rotierenden Klingen anbietet, die sich gegen einen Gitterrost drehen. Die aus Wasser und zerkleinerten Bioabfällen bestehende Suspersion soll in der Hydrolyse- und saure-Gärungs-Stufe eine Verweilzeit von maximal 1 Tag haben. Nach dem Stand der Technik sind auch Hydrolysetemperaturen zwischen 35°C bis 65°C ebenso bekannt wie Temperaturen von 35°C für die mesophile Methangärung oder ca. 55°C für die thermophile Methangärung.

Es ist Aufgabe der vorliegenden Erfindung, das eingangs beschriebene Verfahren zu optimieren und eine hierfür geeignete Vorrichtung anzugeben. Insbesondere sollen das Verfahren und die Vorrichtung eine wirtschaftliche Arbeitsweise bei einfacher kurzzeitiger Handhabung ermöglichen.

Diese Aufgabe wird durch das Verfahren nach Anspruch 1 gelöst. Hierbei werden die Bioabfälle auf Korngrößen von maximal 3 mm, vorzugsweise kleiner 0,5 mm zerkleinert und zu einem homogenen Brei mit einem Trockensubstanzgehalt auf 5 bis 22 %, vorzugsweise 10 %, ggf. durch Wasserzugabe eingestellt, der Brei anschließend einer temperaturgesteuerten sauren aeroben Hydrolyse bei 35°C bis 60°C, vorzugsweise bei 35°C bis 40°C, zugeführt, wobei die Hydrolyse auf maximal 24 h zeitlich begrenzt ist und bei Erreichen von pH-Werten zwischen 3,6 bis 4,5 beendet wird, wonach der Brei einer temperaturgesteuerten Methanisierung bei Temperaturen von 35°C bis 40°C kontinuierlich zugeführt wird, bei der über weniger als 10 Tage die Masse kontinuierlich bei pH-Werten ≥ 7 umgewälzt wird, vorzugsweise mit einer bei einer Drehzahl von ≤ 4 U/min arbeitenden Umwälzvorrichtung, und abschließend die Masse in einen Absetzbehälter zur Trennung der flüssigen und festen Phase überführt wird.

Durch das vorgenannte Verfahren werden die Abfälle vor der Behandlung aufgeschlossen bzw. zerkleinert. Das aufgeschlossene Material wird in einer zweiten Stufe einer aeroben Behandlung unterworfen, wobei aufgrund der hohen Stoffwechselaktivität der aeroben Mikroorganismen ein Teil der organischen Abfallstoffe zu CO₂ verstoffwechselt und ein weiterer Teil mittels von den Mikroorganismen ausgeschiedenen Enzymen in Wasser lösliche, bakteriologisch verstoffwechselbare Verbindungen umgewandelt wird. In der dritten Stufe werden die gelösten organischen Verbindungen durch anaerobe Mikroorganismen zu CO₂ und CH₄ verstoffwechselt.

Vorteilhafte Weiterbildungen des erfindungsgemäßen Verfahrens sind Gegenstand der abhängigen Ansprüche 2 bis 7.

Vorzugsweise wird bei der Hydrolyse- und in der Methanisierungsbehandlung eine gleiche Temperatur eingestellt, wobei auf einheitliche Temperaturregelkreise zurückgegriffen werden kann. Nach einer Ausführungsform des Verfahrens werden 200 Liter der zu behandelnden Bio-Masse aus der Hydrolysierungsstufe über 24 h verteilt in die Methanisierungsstufe überführt. Eine weitere Optimierung ergibt sich durch ständiges Rühren der Masse während der sauren Hydrolyse. Der Gasraum in dem Hydrolysebehälter wird ständig abgesaugt, wobei das abgesaugte Gas zur Vermeidung einer Geruchsbelästigung über einen Kompostfilter ins Freie geleitet wird. Der aus dem Absetzbehälter abgezogene Schlamm wird vorzugsweise in die Methanisie rungsstufe zwecks vollständiger Umsetzung zu Methan und Kohlendioxid zurückgeführt. Das aus dem Reaktor der Methanisierungsstufe abgezogene Biogas wird zumindest teilweise zur Reaktorbegasung verwendet. Es hat sich als optimal erwiesen, wenn die Hydrolyse bei Erreichen von pH-Werten zwischen 3,6 bis 4,5, vorzuzgsweise zwischen 3,8 und 4,5, beendet wird. Nach einer weiteren Verbesserung des erfindungsgemäßen Verfahrens werden die Bioabfälle nach der Zerkleinerung und vor der Hydrolyse einer thermischen, chemischen, enzymatischen oder biologischen Aufschlußbehandlung unterzogen.

Vorrichtungstechnisch wird die obengenannte Aufgabe durch die Vorrichtung mit der Merkmalskombination nach Anspruch 8 gelöst. Als kennzeichnende Teile dieser Vorrichtung sind eine Vibrations-Kolloidmühle zur Zerkleinerung, und/oder ein mit einer Drehschieberpumpe, einer Gasabsaugeinrichtung und eine Heizung ausgestatteter Hydrolysebehälter und/oder ein beheizbarer und begasbarer anaerob arbeitender Scheibenreaktor, der eine mehrere, Scheiben tragende Welle mit einem Drehantriebe besitzt, vorgesehen. Der Scheibenreaktor besteht vorzugsweise aus einem im wesentliche horizontal gelegten Zylinderrohr, in dessen Innenraum längsaxial die Welle drehbar befestigt ist. Nach einer weiteren Ausgestaltung der Erfindung ist jede der Scheiben mit Löchern versehen, wobei zwischen zwei nebeneinander angeordneten Scheiben ein Vlies angeordnet ist, das vorzugsweise aus PE besteht. Dieses Vlies dient als Festbett für Mikroorganismen. Bevorzugt wird der Scheibenreaktorinnenraum zur zusätzlichen Umwälzung mittels Schlauchmembranbelüftern begast.

Weitere Vorteile des erfindungsgemäßen Verfahrens und der Vorrichtung ergeben sich aus der nachfolgenden Beschreibung von Ausführungsbeispielen.

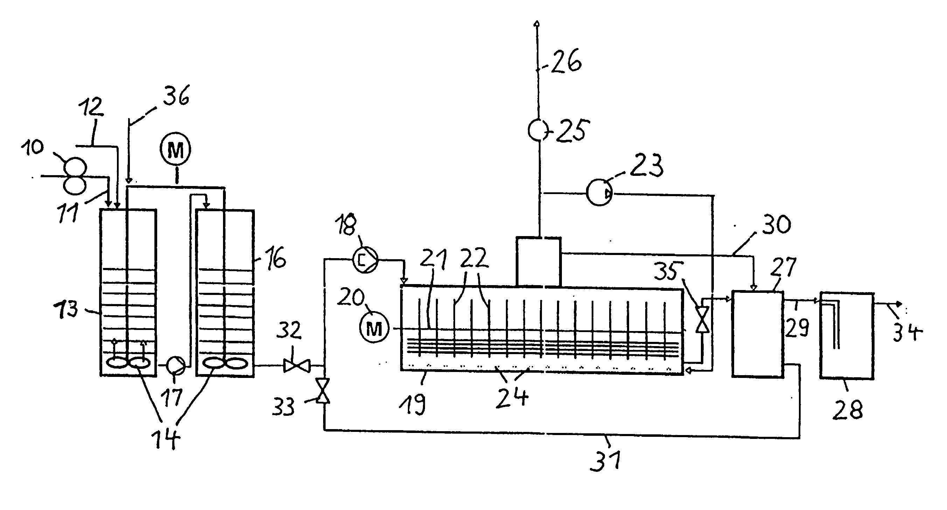

Die einzige Figur zeigt eine schematische Ansicht einer erfindungsgemäßen Vorrichtung.

Die Speisereste aus Großküchen, Restaurantbetrieben, Schnellrestaurants sowie Abfälle aus der Lebensmittelindustrie, aus Schlachthöfen oder Brauereien werden nach Auslese von Stör- und Grobstoffen in einer Vibrations-Kolloidmühle oder einer anderen Zerkleinerungsvorrichtung auf Korngrößen von < 0,5 mm zu einem homogenen Brei zerkleinert. Der derart aufbereitete Brei (siehe Pfeil 11) wird unter Zugabe von Wasser (siehe Pfeil 12) in einen beheizbaren Hydrolysebehälter 13 gegeben. Der Hydrolysebehälter besteht aus einer stehenden Röhre mit einem Durchmesser von 0,5 m und einer Höhe von 1,6 m. Das Material wird durch ein Rührwerk 14 umgewälzt und homogenisiert. Um eine aerobe Hydrolyse zu erreichen, wird z.B. mit einer Drehschieberpumpe durch das Rührwerk die für die optimale Umsetzung erforderliche Luftmenge eingeblasen. Der Hydrolysebehälter wird auf eine Temperatur von 37°C erwärmt. Als Heizung dient z.B. eine Gas-Kesseltherme. Die Temperatur im Hydrolysebehälter wird durch eine Steuereinheit geregelt. Der auf einen Trockensubstanzgehalt von 10 % eingestellte Brei verbleibt maximal 24 h im Hydrolysebehälter, wobei in der genannten Vorrichtung das Füllvolumen von 200 l dem täglichen Anlagendurchsatz entspricht. Der Luftraum im Hydrolysebehälter wird durch einen Verdichter abgesaugt und über einen nicht dargestellten Kompostfilter ins Freie geleitet. Nach erfolgter Hydrolyse wird das breiförmige Material in einen Vorlagebehälter 16 mittels einer Pumpe 17 überführt. Dieser Behälter ist baugleich mit dem Hydrolysebehälter 13 und besitzt ein dementsprechendes Rührwerk 14 sowie eine Belüftung. Durch ständiges Rühren wird ein Absetzen von Feststoffen bzw. die Bildung einer Schwimmschlammschicht vermieden. Die jeweiligen Füllstände in den Behältern 13 und 16 werden durch Schwimmerschalter überwacht.

Mittels einer Pumpe, vorzugsweise Schlauchpumpe 18 wird von dem Vorlagebehälter 16 der Brei in den Scheibenreaktor 19 gepumpt. Eine Steuereinrichtung sorgt dafür, daß das Füllvermögen des Vorlagebehälters (200 l) über 24 h verteilt in den Scheibenreaktor 19 gegeben werden. Dieser Scheibenreaktor wird als Anaerobreaktor betrieben und besteht aus einem 4,0 m langen, liegenden HDPE-Rohr mit einem Durchmesser von 1,0 m. Dieser Reaktor wird zu ca. 2/3, nämlich einer Menge von ca. 2000 l gefüllt. Das in den Reaktorbehälter gepumpte Material verbleibt dort etwa 10 Tage. Der Scheibenreaktor 19 besitzt eine mittels eines Motors 20 angetriebene Welle 21, die mehrere (hier 15 dargestellte) in Sandwich-Bauweise ausgebildete, HDPE-Lochscheiben trägt. Zwischen jeweils zwei Scheiben befindet sich ein PE-Vlies, das als Festbett für Mikroorganismen dient. Die Welle 21 wird mit einer niedrigen Umdrehungsgeschwindigkeit von maximal 4 U/min gedreht, womit gewährleistet ist, daß die sich festgesetzten Organismen nicht aufgrund von Scherkräften abgespült werden können.

Das aus dem Reaktor abgezogene Biogas wird zum Teil mittels eines Kompressors 23 Begasungseinheiten 24 zugeführt, die sich im unteren Bereich des Reaktors 19 befinden. Das nicht rückgeführte Biogas wird mittels einer Gasuhr 25 gemessen und einer Biogasverwertung (siehe Pfeil 26) zugeführt.

Ziel des Reaktor-Begasens ist zum einen eine Unterstützung der Umwälzung der im Reaktor befindlichen Biomasse und dient zum anderen zur Vermeidung von Schlammabsetzungen. Im Reaktor wird eine Pfropfenströmung erzielt, wobei das zwischen die Scheiben 22 eingeblasene Gas wie ein Vorhang wirkt. Die Temperatur im Reaktor wird durch eine entsprechende Heizung auf 37°C geregelt. Zur Beheizung können ebenso wie beim Hydrolysebehälter eingesetzte Gas-Kesseltherme verwendet werden. Das Heizgas wird durch jeweilige Heizrohre über eine geeignete Ventilsteuerung automatisch in den Hydrolysebehälter 13, den Vorlagebehälter 16 und den Scheibenreaktor 19 geleitet. Die Beheizung des Scheibenreaktors hat in jedem Fall Priorität, da die anaeroben Mikroorganismen anfälliger auf Temperaturschwankungen reagieren.

Das Ablaufwasser aus dem Scheibenreaktor 19 wird über einen natürlichen Ablauf in zwei Absetzbehälter 27 und 28, beispielsweise 220 l/PE-Behälter abgeleitet. Die Behälter sind im oberen Bereich durch eine Schlauchleitung 29 verbunden, so daß sich im ersten Behälter 27 vorhandener Feststoff absetzen und überstehendes Wasser über den Ablauf 29 und ein Tauchrohr in das zweite Faß 28 gelangen kann. Der erste Behälter 27 ist durch eine Gasleitung 30 mit dem Scheibenreaktor 19 verbunden, so daß auch dort die anaeroben Verhältnisse gehalten werden. Über eine Schlauchpumpe wird über die Leitung 31 stündlich eine definierte Menge des im Behälter 27 abgesetzten Schlammes zurückgeführt. Zur Beschickung des Scheibenreaktors aus dem Vorlagebehälter sowie aus dem Auffangbehälter werden regelbare Ventile 32 und 33 verwendet. Das aus dem zweiten Auffangbehälter 28 ableitbare Wasser wird einer Abwasserbehandlung 34 zugeführt.

Eine geeignete Meß-, Regel- und Steuerungstechnik dient dazu, die Temperatur im Hydrolysebehälter, im Vorlagebehälter und im Scheibenreaktor konstant auf 37°C einzustellen. Ferner wird nach Maßgabe einer Messung der Sauerstoffkonzentration in der Suspension die Luftzufuhr im Hydrolysereaktor 13 geregelt. Die biologischen Abbauprozesse im Hydrolysereaktor können über CO₂-Konzentrationsmessungen in der Abluft sowie über den pH-Wert in der Suspension gemessen werden.

Eine Steuerung der Pumpen und Ventile 32, 33 und 35 sorgt für eine optimale Beschickung bzw. Rückführung in gewünschten dosierten Mengen.

Die vorbeschriebene Vorrichtung arbeitet folgendermaßen:

Angelieferte Bioabfälle werden zunächst auf genannte Korngrößen zerkleinert und durch Wasserzugabe auf einen Trockensubstanzgehalt von ca. 10 % eingestellt. Unter gesteuerter Luftzugabe wird die im Hydrolysebehälter befindliche Suspension aerob gehalten, wobei die Sauerstoff-Konzentration über 2 mg/l Suspension eingestellt wird. Das im Hydrolysebehälter befindliche Material wird innerhalb der Aufenthaltsdauer von 24 h mehr oder weniger weitgehend durch mikrobielle oder autolytische Aktivitäten enzymatisch in wasserlösliche Verbindungen überführt. Durch diese Verbindungen, u.a. Essigsäure, fällt der pH-Wert in der Suspension auf Werte um 4. Nach maximal 24 h wird die Suspension abgepumpt und in den Vorlagebehälter 16 zur Zwischenlagerung überführt. Ggf. kann aus der Suspension noch nicht umgesetztes Material entzogen und erneut der Hydrolysestufe zugeführt werden.

Aus dem Vorlagebehälter 16 wird kontinuierlich die dort zwischengelagerte Suspension in die Methanisierungsstufe, d.h. den Scheibenreaktor, überführt. In dem anaerob arbeitenden Scheibenreaktor 19 wird die wassergelöste Organik sowie die noch vorhandene feinpartikuläre Festorganik zu Biogas umgesetzt. Die Beschickung des Scheibenreaktors 19 wird in Abhängigkeit des im Scheibenreaktors gemessenen pH-Wertes vorgenommen, um eine Versäuerung der Anaerobstufe im Scheibenreaktor 19, der möglichst bei pH-Werten ≥ 7 betrieben werden soll, zu verhindern. Bei einem Absinken unter den pH-Wert 7 droht ansonsten eine stark überschäumende Reaktion unter Freisetzung hoher Kohlendioxid-Konzentrationen.

Die im wesentlichen zweistufige Verfahrenstechnik (saure Hydrolyse und Methanisierung) führt dazu, daß beide Stufen örtlich getrennt betrieben werden können bzw. das bereits hydrolysierte Suspensionen gesteuert einer anaeroben Methanisierungsstufe beigemengt werden können. Durch Hydrolyse gelöste organische Anteile sowie etwa noch vorhandene feinstpartikuläre Bestandteile werden jedenfalls in der anaeroben Methanisierungsstufe deutlich schneller abgebaut als nicht behandeltes Material. Hydrolysiertes biogenes Material kann auf diese Art durchaus 10-fach schneller abgebaut als unter rein anaeroben Bedingungen. Durch die gewählte Prozeßsteuerung wird insbesondere eine massive Nachversäuerung der anaeroben Stufe vermieden.

Vorteilhafte Verwendungen des beschriebenen Verfahrens bzw. der betreffenden Vorrichtung bieten sich in Urlaubsgebieten in der Hochsaison an, die durch intensiven Fremdenverkehr stark überlastet sind. In Gastronomiebetrieben fehlen in der Regel Fettabscheider, wodurch große Mengen extrahlerbarer Stoffe, wie beispielsweise Fette, Öle etc., in Kläranlagen zu gelangen drohen. Ohne die erfindungsgemäße Verfahrenstechnik drohen Geruchsbelästigungen, Schaumbildung an der Oberfläche von Belebungsbecken, Schlammabtrieb aus Nachklärbecken und Fettablagerungen in den Kanälen sowie in vorhandenen Leitungssystemen. Eine Vorbehandlung der Speisereste solcher Gastronomiebetriebe ist zwingend geboten. Entscheidend sind beim Abbau von Speiseresten der Verlauf und die Geschwindigkeit der Stoffwechselprozesse sowie der durch Mikroorganismen erzielbare Energiegewinn.

Der aerobe Abbau von organischen Stoffen wird von einer komplexen Mikrobenpopulation durchgeführt. Dabei sind einzelne Organismenarten in der Lage, den Abbau vom Ausgangsprodukt zum Endprodukt vorzunehmen. Bis zu 50 % des in der Biomasse enthaltenen Kohlenstoffes werden bei diesem Abbau als bakterielle Biomasse (Schlamm) festgelegt. Die Generationszeiten der meisten aeroben Mikroorganismen liegen im Bereich von wenigen Minuten bis maximal 24 h.

Der Abbau im anaeroben Milieu verläuft in vier Stufen, die zum Teil von verschiedenen Organismengruppen durchgeführt werden. An diesen vier Stufen, die alle gleichzeitig ablaufen können, sind ausnahmslos Bakterien beteiligt. Die hochmolekularen Stoffe, wie Proteine, Fette, Polysaccharide, Nukleinsäuren u.a., werden, wie auch beim aeroben Abbau, zunächst durch extracelluläre Enzyme in ihre niedermolekularen Bestandteile zerlegt (Hydrolyse) und von den Organismen aufgenommen. Die gebildeten Mono- und Dimere können von den gleichen Bakterien wie bei der Hydrolyse aufgenommen und weiter abgebaut werden (Säurebildung). Den Hauptanteil der Produkte machen die Carboxylsäuren, wie Acetat, Propionat und Butyrat, aus. Dazu kommen noch Milchsäure und Alkohol. Zu kleineren Teilen werden auch noch Wasserstoff und Kohlendioxid gebildet. Die Konzentration des intermediär gebildeten Wasserstoffes bestimmt die Art der Produkte. Je höher der Partialdruck von H₂ ist, um so weniger reduzierte Produkte wie Essigsäuren und Wasserstoff werden gebildet. Bei zu starker Ansäuerung, d.h. wenn die produzierten Säuren nicht weiter verwendet werden, findet die Hemmung dieser Organismen (insbesondere bei pH<4) statt. In der nächsten Phase werden die in der Vergärungsstufe gebildeten organischen Säuren und Alkohole zu Essigsäure, Wasserstoff und Kohlendioxid umgewandelt (Essigsäurebildung). Es handelt sich hierbei um den thermodynamisch schwierigsten Schritt des Gesamtabbaues. Diese Reaktionen können nur dann exotherm ablaufen, wenn der Wasserstoffpartialdruck sehr niedrig ist. Danach werden in der vierten Stufe (Methanbildung) die Essigsäure und das Kohlendioxid mit molekularem Wasserstoff zu Methan umgesetzt. Die Umsetzung wird von obligat anaeroben Bakterien durchgeführt.

Für einen prozeßstabilen Verlauf des anaeroben Abbaues ist es von Bedeutung, welcher der vier Abbauschritte bei bestimmten Prozeßzuständen den geschwindigkeitslimitierenden Schritt darstellt. Die Hydrolysephase ist dann als umsatzlimitierender Abbauschritt anzusehen, wenn das Substrat sich aus überwiegend höhermolekularen Verbindungen zusammensetzt oder als Mischsubstrat vorliegt, d.h. einen hohen Anteil an Lignin und Cellulose besitzt. Da man davon ausgeht, daß in den Speiseresten überwiegend Fette, Proteine und Kohlenhydrate enthalten sind, gelten vornehmlich die Acetogenese und Methanogenese als geschwindigkeitsbestimmend. Die Generationszeiten der meisten anaeroben Mikroorganismen liegen im Bereich von 10 bis 24 Tagen und mehr.

Der biologische Abbau der organischen Substanz erfolgt sowohl im aeroben wie im anaeroben Milieu durch enzymkatalysierte Stoffwechselprozesse der Mikroorganismen. Die für den Abbauprozeß erforderlichen substratspezifischen Enzyme sind entweder in der Biomasse bereits vorhanden - so für leicht abbaubare Substanzen - oder werden von entsprechenden Organismenarten synthetisiert; letzteres trifft vor allem bei schwer und langsam abbaubaren Substraten zu. Die Prozeßgeschwindigkeit wird dabei von der Zusammensetzung und der Menge des abzubauenden Substrates sowie dem Gehalt der substratspezifischen Enzyme der Organismen bestimmt. Die Abhängigkeit der Umsatzgeschwindigkeit von der Substratkonzentration verhält sich derart, daß bei niedrigen Substratkonzentrationen geringe Umsatzgeschwindigkeiten zu erwarten sind, die bei hohen Substratkonzentrationen ansteigen. Ab einer systemspezifischen Grenzkonzentration läßt sich keine weitere Erhöhung der Umsatzgeschwindigkeit mehr erzielen.

Der Energiegewinn der Organismen erfolgt durch die Abspaltung von Wasserstoff aus dem Nährsubstrat als H-Donator und dessen Übertragung auf einen anderen Stoff, dem H-Acceptor. Der bei schrittweisem Abbau abgespaltene Wasserstoff wird dabei auf Nicotinsäureamid-Adenin-Dinukleotid übertragen (NAD → NADH₂). Der universelle Überträger der Energie ist das Adenosintriphosphat (ATP), eine energiereiche Verbindung mit drei Phosphatresten. Bei der hydrolytischen Abspaltung eines Phosphatrestes werden 29 kJ/mol abgebautem Substrat an Energie frei (Phosphorylierung). Die Höhe des möglichen Energiegewinnes der Organismen ist abhängig von der Höhe der Differenz zwischen den Energiegehalten von Ausgangs- und Endprodukt.

Am Beispiel des biologischen Glucoseabbaues lassen sich Gemeinsamkeiten und Unterschiede des aeroben und anaeroben Abbaues aufzeigen. Bis zur Bildung des Intermediärproduktes Pyruvat, das sowohl bei aerobem als auch bei anaerobem Stoffwechselprozeß entsteht, unterscheiden sich die aeroben und anaeroben Abbauwege nicht.

Bei aerobem Abbau folgen dann die Teilschritte oxydative Decarboxylierung, Tricarboncyklus und Atmungskette. Dabei dient Sauerstoff als terminaler Wasserstoffacceptor; in der Atmungskette wird Wasserstoff in einer biochemischen Knallgasreaktion zu Wasser oxidiert (Atmungskettenphosphorylierung). Als Endprodukt werden die energiearmen anorganischen Stoffe CO₂ und H₂O gebildet. Der Energiegewinn der Mikroorganismen bei aerobem Abbau ist groß.

Im anaeroben Milieu steht kein Sauerstoff als H-Acceptor zur Verfügung. Es müssen daher Stoffwechselzwischenprodukte als H-Acceptor fungieren (Substratphosphorylierung). Diese werden durch die unterschiedlichen Organismen auf vielfältige Weise erzeugt und anschließend aus dem Zellinneren ausgeschleust (Alkohole, Fettsäuren, niedere organische Säuren). Ein Teil dieser Produkte kann dann von anderen Bakteriengruppen weiter zu Kohlendioxid und Methan umgesetzt werden. Aufgrund der bei anaerobem Abbau fehlenden Atmungskettenphosphorylierung ist die Energiedifferenz zwischen Ausgangs- und Endprodukten in anaerobem Milieu nur gering, d.h. der Energiegewinn der Mikroorganismen ist entscheidend niedrig. Die noch energiereichen Produkte des anaeroben Abbaues - organische Stoffwechselprodukte und Methan - können in aerobem Milieu biologisch weiter abgebaut werden bzw. durch Verbrennung in Heizkesseln oder Gasmotoren genutzt werden.

Die bei aerobem Abbau freigesetzte Energie wird durch Bildung von 38 ATP/mol Glucose verwertet, wobei 34 ATP/mol alleine durch die Atmungskette gebildet werden. Zusätzlich zu den in 38 ATP/mol gespeicherter, im biologischen Prozeß unmittelbar nutzbaren Energie, entsprechend 1100 kJ/mol, werden 1770 kJ/mol (= 60 %) als Wärmeenergie freigesetzt. Dadurch würde es bei aerobem Abbau von Speiseresten, dessen entsprechende Konzentration vorausgesetzt, zu einer Selbsterwärmung der Speisereste bis in den thermophilen Temperaturbereich kommen.

Der Energiegewinn der Mikroorganismen bei anaerobem Abbau beträgt dagegen nur 2 ATP/mol Glucose, das sind lediglich ca. 5 % des bei aerobem biologischem Glucoseabbau möglichen Energiegewinnes. Dementsprechend wird im biologischen anaeroben Prozeß auch nur wenig Wärmeenergie freigesetzt, die für eine signifikante Selbsterwärmung der Speisereste nicht ausreicht. Im anaeroben Prozeß bleibt ein Großteil der Energie in den organischen Zwischen- und Endprodukten bzw. im Methan des Biogases erhalten.

In einer Energiegesamtbilanz zeigt ein Vergleich, daß die im aeroben und anaeroben Abbau in Form von Wärmeenergie und Biogas insgesamt freigesetzte Energie, bezogen auf den abgebauten CSB, für beide Prozeßvarianten das gleiche Ergebnis aufweist.

Die vorstehende Erfindung schafft erstmals die Möglichkeit, die Stoffwechselprozesse im Hinblick auf eine kurze Aufenthaltszeit bei gleichzeitiger Gewährleistung stabiler Prozeßbedingungen zu optimieren.

## Patentansprüche

1. Verfahren zur Behandlung von strukturfreien oder strukturarmen Bioabfällen, nämlich Speiseresten und/oder Rückständen aus Abfällen aus Haushalten, der Gastronomie sowie der Lebensmittelindustrie, die nach ihrer Zerkleinerung und Homogenisierung einer weitgehend aeroben Hydrolyse unterworfen werden und danach einer anaeroben Methanisierung zugeführt werden, bei der das entstehende Biogas abgezogen wird, wobei die Bioabfälle auf Korngrößen von maximal 3 mm, vorzugsweise < 0,5 mm, zerkleinert und zu einem homogenen Brei mit einem Trockensubstanzgehalt auf 5 bis 22 %, vorzugsweise 10 %, ggf. durch Wasserzugabe eingestellt werden, der Brei anschließend einer temperaturgesteuerten, auf maximal 24 h zeitlich begrenzien, sauren Hydrolyse bei 35°C bis 60°C, vorzugsweise bei 35°C bis 40°C, zugeführt wird und die Hydrolyse bei Erreichen von pH-Werten zwischen 3,6 bis 4,5 beendet wird, wonach der Brei einer temperaturgesteuerten Methanisierung bei Temperaturen von 35°C bis 40°C kontinuierlich zugeführt wird, bei der über weniger als 10 Tage die Masse kontinuierlich bei pH-Werten ≥ 7 umgewälzt wird, vorzugsweise mit einer bei einer Drehzahl von ≤ 4 U/min arbeitenden Umwälzvorrichtung, und abschließend die Masse in einen Absetzbehälter zur Trennung der flüssigen und festen Phase überführt wird.

2. Verfahren nach Anspruch 1, **dadurch gekennzeichnet, daß** bei der Hydrolyse und in der Methanisierungsbehandlung gleiche Temperaturen eingestellt werden.

3. Verfahren nach Anspruch 1 oder 2, **dadurch gekennzeichnet, daß** die Masse aus der Hydrolysierungsstufe in einer Menge von 200 l über 24 h verteilt in die Methanisierungsstufe überführt wird.

4. Verfahren nach einem der Ansprüche 1 bis 3, **dadurch gekennzeichnet, daß** die Masse während der Hydrolyse ständig gerührt wird und/oder daß die Hydrolyse bei Erreichen von pH-Werten zwischen 3,8 bis 4,5, beendet wird.

5. Verfahren nach einem der Ansprüche 1 bis 4, **dadurch gekennzeichnet, daß** der Gasraum in dem Hydrolysebehälter ständig abgesaugt und das abgesaugte Gas über einen Kompostfilter ins Freie geleitet wird.

6. Verfahren nach einem der Ansprüche 1 bis 5, **dadurch gekennzeichnet, daß** der aus dem Absetzbehälter abgezogene Schlamm teilweise in die Methanisierungsstufe zurückgeführt wird und/oder daß das aus dem Reaktor der Methanisierungsstufe abgezogene Biogas zumindest teilweise zur Reaktorbegasung verwendet wird.

7. Verfahren nach einem der Ansprüche 1 bis 6, **dadurch gekennzeichnet, daß** die Bioabfälle nach der Zerkleinerung und vor der Hydrolyse einer thermischen, chemischen, enzymatischen oder biologischen Aufschlußbehandlung unterzogen werden.

8. Vorrichtung zur Durchführung des Verfahrens nach einem der Ansprüche 1 bis 7, die eine Zerkleinerungsvorrichtung, eine Mischvorrichtung, einen belüftbaren Hydrolysebehälter (13), einen Biogas-Reaktor, einen Absetzbehälter und jeweilige Materialfördereinrichtungen zur Überführung des Bioabfalles in die nächste Stufe bzw. Rückführung in eine vorherige Stufe aufweisen, **gekennzeichnet durch** mindestens eine der nachfolgenden Vorrichtungen:
a) eine Vibrations-Kolloidmühle zur Zerkleinerung,
b) ein mit einer Drehschieberpumpe, einer Gasabsaugeinrichtung und einer Heizung ausgestatteter Hydrolysebehälter (13) und
c) ein beheizbarer und begasbarer anaerob arbeitender Scheibenreaktor (19), der eine mehrere Scheiben (22) tragende Welle (21) mit einem Drehantrieb (20) besitzt.

9. Vorrichtung nach Anspruch 8, **dadurch gekennzeichnet, daß** der Scheibenreaktor (19) aus einem im wesentlichen horizontal gelegten Zylinderrohr besteht, in dessen Innenraum längsaxial die Welle drehbar befestigt ist und/oder daß jede der Scheiben (22) mit Löchern versehen ist und daß zwischen zwei nebeneinander angeordneten Scheiben (22) ein Vlies, vorzugsweise aus PE, als Festbett für Mikroorganismen angeordnet ist.

10. Vorrichtung nach einem der Ansprüche 8 oder 9, **dadurch gekennzeichnet, daß** der Scheibenreaktor-Innenraum mittels Schlauchmembranbelüftern (24) begasbar ist.

## Revendications

1. Procédé de traitement de déchets biologiques exempts de structures ou pauvres en structures, à savoir de restes de repas et/ou de résidus d'ordures en provenance de ménages, de la gastronomie ainsi que de l'industrie alimentaire, qui - après leur broyage et homogénéisation - sont soumis à une hydrolyse dans une large mesure aérobie et qui sont amenés ensuite à une méthanisation anaérobie dans laquelle le biogaz produit est évacué, les déchets biologiques étant broyés de manière à présenter une granulométrie de 3 mm au maximum, de préférence inférieure à 0,5 mm, et étant conditionnés de manière à former une pâte homogène présentant une teneur en substance sèche de 5 à 22 %, de préférence de 10 %, le cas échéant en ajoutant de l'eau, ladite pâte étant amenée ensuite à une hydrolyse acide à température contrôlée, à des températures comprises entre 35°C et 60°C, de préférence entre 35°C et 40°C, l'hydrolyse étant limitée à 24 heures au maximum et étant terminée lorsque des valeurs pH comprises entre 3,6 et 4,5 sont atteintes, après quoi la pâte est amenée de façon continue, à des températures comprises entre 35°C et 40°C, à une méthanisation à température contrôlée dans laquelle la masse est retournée continûment à des valeurs pH de ≥ 7 durant moins de 10 jours, de préférence par l'intermédiaire d'un dispositif à retourner travaillant à une vitesse de rotation de ≤ 4 tours/minute, et, finalement, la masse étant transférée dans un réservoir de séparation destiné à séparer les phases liquide et solide.

2. Procédé selon la revendication 1, **caractérisé par le fait que** l'on règle les mêmes températures durant l'hydrolyse et dans le traitement de méthanisation.

3. Procédé selon la revendication 1 ou 2, **caractérisé par le fait que** la masse provenant de l'étape d'hydrolysation est amenée à l'étape de méthanisation en une quantité de 200 l, répartie sur 24 heures.

4. Procédé selon l'une des revendications 1 à 3, **caractérisé par le fait que**, durant l'hydrolyse, la masse est agitée sans cesse et/ou que l'hydrolyse est terminée lorsque des valeurs pH comprises entre 3,8 et 4,5 sont atteintes.

5. Procédé selon l'une des revendications 1 à 4, **caractérisé par le fait que** l'espace à gaz dans le réservoir d'hydrolyse est évacué de manière permanente et que le gaz évacué s'échappe dans l'atmosphère à travers un filtre de compost.

6. Procédé selon l'une des revendications 1 à 5, **caractérisé par le fait que** la boue extraite du réservoir de séparation est ramenée en partie à l'étape de méthanisation et/ou que le biogaz évacué du réacteur de l'étape de méthanisation est utilisé au moins en partie pour alimenter en gaz le réacteur.

7. Procédé selon l'une des revendications 1 à 6, **caractérisé par le fait que**, après le broyage et avant l'hydrolyse, les déchets biologiques sont soumis à un traitement thermique, chimique, enzymatique ou biologique de décomposition.

8. Dispositif destiné à la mise en oeuvre du procédé selon l'une des revendications 1 à 7, qui présente un dispositif à broyer, un dispositif mélangeur, un réservoir d'hydrolyse (13) qui peut être aéré, un réacteur à biogaz, un réservoir de séparation et des dispositifs respectifs de transport de matière pour amener les déchets biologiques à l'étape suivante ou bien pour les ramener à une étape précédente, **caractérisé par** au moins un des dispositifs suivants :
a) un moulin colloïdal vibrant pour le broyage
b) un réservoir d'hydrolyse (13) qui est équipé d'une pompe à tiroirs rotatifs, d'un dispositif à aspirer le gaz et d'un chauffage, et
c) un réacteur à disques (19) qui possède un arbre (21) portant plusieurs disques (22) et pourvu d'un entraînement rotatif (20), ledit réacteur à disques pouvant être chauffé, alimenté en gaz et travaillant de manière anaérobie.

9. Dispositif selon la revendication 8, **caractérisé par le fait que** le réacteur à disques (19) se compose d'un tube cylindrique qui est disposé pour l'essentiel horizontalement et à l'intérieur duquel l'arbre est monté à rotation dans la direction de l'axe longitudinal et/ou que chacun des disques (22) est percés de trous et que, entre deux disques (22) disposés l'un à côté de l'autre, est disposé un non-tissé, de préférence en polyéthylène, en tant que lit solide pour les microorganismes.

10. Dispositif selon l'une des revendications 8 ou 9, **caractérisé par le fait que** l'intérieur du réacteur à disques peut être alimenté en gaz au moyen d'aérateurs à membranes tubulaires (24).

## Claims

1. Method of treating unstructured or loosely structured organic wastes, namely, food residues and/or residues of household waste, from gastronomic facilities as well as the food industry, in which after comminution and homogenization, the waste is subjected to a largely aerobic hydrolysis and then to an anaerobic methanization from which the arising biogas is withdrawn, wherein the organic wastes are comminuted to a particle size of at most 3 mm preferably less than 0,5 mm and is brought to a homogeneous slurry with a dry substance content of 5 to 22%, preferably 10%, optionally with addition of water, the slurry is then fed to a controlled temperature acid-hydrolysis 35°C to 60°C, preferably 35°C to 40°C and the hydrolysis is terminated upon reaching of pH values between 3,6 and 4,5, after which the slurry is continuously fed to a controlled temperature methanization at temperatures of 35 to 40°C in which over less that 10 days the mass is continuously turned over at pH values ≥ 7, preferably with a circulating device rotating at a speed of ≤ 4 RPM and then the mass is transferred to a settling tank for separating the liquid and solid phase.

2. Method according to claim 1, **characterized in that** the hydrolysis and the methanization treatment are adjusted to the same temperature.

3. Method according to claim 1 or 2, **characterized in that** the mass is transferred from the hydrolysis stage and into the methanization stage in an amount of 200 l distributed over 24 hours.

4. Method according to claim 1 to 3, **characterized in that** the mass is continuously stirred during the hydrolysis and/or that the hydrolysis is terminated upon reaching of a pH values between 3,8 to 4,5.

5. Method according to claim 1 to 4, **characterized in that** the gas space in the hydrolysis vessel is continuously evacuated and the evacuated gas is released into the atmosphere through a compost filter.

6. Method according to claim 1 to 5, **characterized in that** the sludge withdrawn from the settling thank is partly recycled to the methanization stage and/or that the biogas withdrawn from the reactor of the methanization stage is at least partly used for reactor gasification.

7. Method according to claim 1 to 6, **characterized in that** the organic wastes after comminution and prior to hydrolysis are subject to a thermal, chemical or biological degradation treatment.

8. Apparatus for carrying out the method according to one of the claims 1 to 7 which has a comminution device, a mixing device, an aeratable hydrolysis vessel (13), a biogas reactor, a settling tank and respective material forwarding devices for transferring the organic wastes into the successive stage or for recycling it into a preceding stage, **characterized by** at least one of the following devices:
a) a vibration colloid mill for comminution,
b) a hydrolysis vessel (13) equipped with a rotary disk pump, a gas suction device and a heating system and/or
c) a heatable and gasifiable anaerobically operating disk reactor (19) which has a shaft (21) with a plurality of disks (22) and which is provided with a rotary drive (20).

9. Apparatus according to claim 8, **characterized in that** the disk reactor (19) has a substantially horizontally oriented cylinder tube in whose interior space the shaft is oriented longitudinally and is mounted for rotation and/or that each of the disks (22) is provided with holes and that between two neighboring disks (22) a fleece preferably or polyethylene is arranged as a solid bed for mircoorganisms.

10. Apparatus according to one of claims 8 or 9, **characterized in that** the interior of the disk reactor is gasifiable my means of a hose membrane aerator (24).
